# EUROPEAN PATENT SPECIFICATION

(11) **EP 3 296 390 B1**
(45) Date of publication and mention of the grant of the patent: **04.01.2023**
(21) Application number: 16780120.8
(22) Date of filing: 14.04.2016
(51) Int. Cl.: C12N 5/071, C12N 5/0797

(54) **METHOD FOR PRODUCING STEM CELL CLONES SUITABLE FOR INDUCTION OF DIFFERENTIATION INTO SOMATIC CELLS**
VERFAHREN ZUR HERSTELLUNG VON STAMMZELLKLONEN ZUR INDUZIERUNG DER DIFFERENZIERUNG IN KÖRPERZELLEN
PROCÉDÉ DE PRODUCTION DE CLONES DE CELLULES SOUCHES CONVENANT POUR L'INDUCTION D'UNE DIFFÉRENCIATION EN CELLULES SOMATIQUES

(30) Priority: 14.04.2015 JP 2015082768
(43) Date of publication of application: 21.03.2018
(73) Proprietor: Kyoto University, Kyoto-shi, Kyoto 606-8501 (JP); Megakaryon Corporation, Kyoto-shi, Kyoto 606-8305 (JP)
(72) Inventor: ETO, Koji, Kyoto-shi Kyoto 606-8501 (JP); ENDO, Hiroshi, Kyoto-shi Kyoto 606-8501 (JP); SHIGEMORI, Tomohiro, Kyoto-shi, Kyoto 600-8815 (JP)
(74) Representative: MacLean, Martin Robert
(86) International application number: PCT/JP2016/062040
(87) International publication number: WO 2016/167329

(56) References cited:
- WO-A1-2011/052504
- WO-A1-2011/096482
- WO-A1-2014/123242
- JP-A- 2013 240 308
- US-A1- 2012 009 158
- KEISUKE OKITA ET AL: "A more efficient method to generate integration-free human iPS cells", NATURE METHODS, vol. 8, no. 5, 1 May 2011 (2011-05-01), pages 409-412, XP055176852, ISSN: 1548-7091, DOI: 10.1038/nmeth.1591
- KEISUKE OKITA ET AL: "An Efficient Nonviral Method to Generate Integration-Free Human-Induced Pluripotent Stem Cells from Cord Blood and Peripheral Blood Cells", STEM CELLS., vol. 31, no. 3, 25 February 2013 (2013-02-25), pages 458-466, XP055461400, US ISSN: 1066-5099, DOI: 10.1002/stem.1293
- NAKAMURA, SOU ET AL.: 'Expandable Megakaryocyte Cell Lines Enable Clinically Applicable Generation of Platelets from Human Induced Pluripotent Stem Cells' CELL STEM CELL vol. 14, 2014, pages 535 - 548, XP055308116

## Description

### Technical Field

The present invention relates to, for instance, a method for producing a cell population derived from a single cell, namely cloning, of stem cells or somatic cells composed of various populations by using reprogramming of somatic cells.

### Background Art

Cloning is an important step in the development of cell lines, and this cloning has conventionally been carried out using the limiting dilution method.

Although cells are converted to a desired cell type by introducing an exogenous gene into the cells (Patent Document 1, Non-Patent Documents 1 and 2), the resulting cells are not uniform due to such factors as differences in the number of copies of the introduced exogenous gene or differences in the introduced site in the chromosome. Therefore, although cloning is thought to be able to be carried out according to the limiting dilution method, the limiting dilution method cannot necessarily be applied to all cells.

In addition, since cells obtained by introducing an exogenous gene have a low probability of allowing desired cells to be obtained again even if the cells are attempted to be obtained by the same method, in the case of the cells requiring gene manipulation such as homologous recombination, there are limitations on those cells types that permit such manipulation.

Although replacement therapy has been proposed that involves inducing reconversion to T lymphocytes from iPS cells obtained by reprogramming T-lymphocytes retaining a desired TCR type (Patent Document 2 or Non-Patent Document 3), this therapy is not conducted for the purpose of cloning or gene manipulation.

WO2014/123242A1 describes production methods for megakaryocytes and platelets. Okita et al. (Nat Methods. 2011 May;8(5):409-12) describes a method for generating integration-free human iPS cells. Okita et al. (Stem Cells. 2013 Mar;31(3):458-66) describes a nonviral method to generate integration-free human-induced pluripotent stem cells from cord blood and peripheral blood cells. US2012/0009158A1 describes methods to generate a secondary iPS cell to produce somatic cells of a rare differentiation cell type fate. Nakamura et al. (Cell Stem Cell. 2014 Apr 3;14(4):535-48) describes that expandable megakaryocyte cell lines enable clinically applicable generation of platelets from human induced pluripotent stem cells. WO2011/052504A1 describes a method for induction of differentiation of stem cells into hepatocytes.

### Citation List

### Patent Documents

Patent Document 1: WO 2014/148646
Patent Document 2: WO 2011/096482

### Non-Patent Documents

Non-Patent Document 1: Nakamura S, et al, Cell Stem Cell. 14:535-548, 2014
Non-Patent Document 2: Tanaka A, et al, PLoS One. 8:e61540, 2013
Non-Patent Document 3: Nishimura T, et al., Cell Stem Cell. 12(1):114-126, 2013

### Summary

### Technical Problem

An object of the present invention is to produce a cell population derived from a single cell, namely cloning, of stem cells or somatic cells composed of various populations.

### Solution to Problem

When the inventors of the present invention isolated stem cells having a gene associated with inducing differentiation into somatic cells incorporated in a chromosome thereof from a stem cell colony prepared by one or multiple rounds of dedifferentiation, the isolated stem cells were found to be suitable for inducing differentiation into somatic cells, thereby leading to completion of the present invention.

The present invention is defined by the claims.

There is provided a method for producing a stem cell clone according to claim 1.

The differentiation induction efficiency of isolated stem cell clones into somatic cells may be higher in comparison with that of stem cells prior to cloning.

The somatic cells are defined in the claims.

The exogenous gene associated with induction of differentiation is at least one exogenous gene selected from the group consisting of oncogenes including MYC family genes, genes (polycomb genes) inhibiting expression of p16 gene or p19 gene including Bmi1, and apoptosis suppressing genes including BCL-XL gene.

Stem cells expressing MEG3 may be isolated.

The exogenous gene associated with induction of differentiation may be functionally linked to a drug-responsive promoter.

The dedifferentiation in step (iii) is carried out by introducing a reprogramming factor selected from the group consisting of OCT3/4, SOX2 and KLF4.

A method for producing megakaryocyte progenitor cells is defined by claim 5.

A method for producing platelets is defined by claim 6.

The produced platelets may be deficient in HLA.

### Advantageous Effects of Invention

According to the present invention, a stem cell clone can be produced, as claimed, that is suitable for inducing differentiation into somatic cells defined by the claims. In addition, stem cells cloned according to the present invention have superior proliferation potency in comparison with stem cells cloned according to the conventional limiting dilution method. For example, not only do secondary megakaryocyte progenitor cell clones prepared in accordance with the present invention have superior cell proliferation potency and ability to mature into megakaryocytes in comparison with conventional clones, megakaryocytes prepared from these clones have a high platelet production capacity.

### Brief Description of Drawings

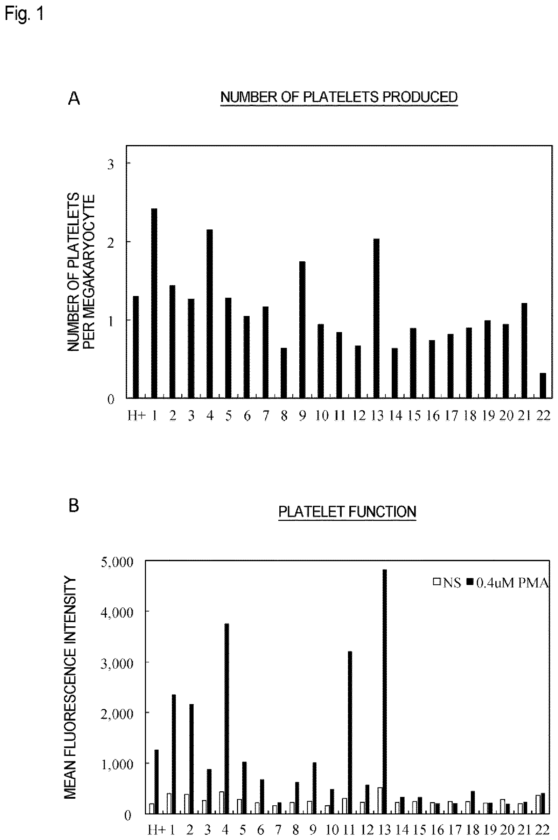

Fig. 1 is a graph indicating the numbers of platelets produced derived from 22 megakaryocyte progenitor cell clone candidates obtained by the limiting dilution method along with megakaryocyte progenitor cells (H+) composed of various original populations (Fig. 1A), and the fluorescence intensity in lieu of the amount of GPIIb/IIIa complex formed by PMA stimulation of the platelets (Fig. 1B).

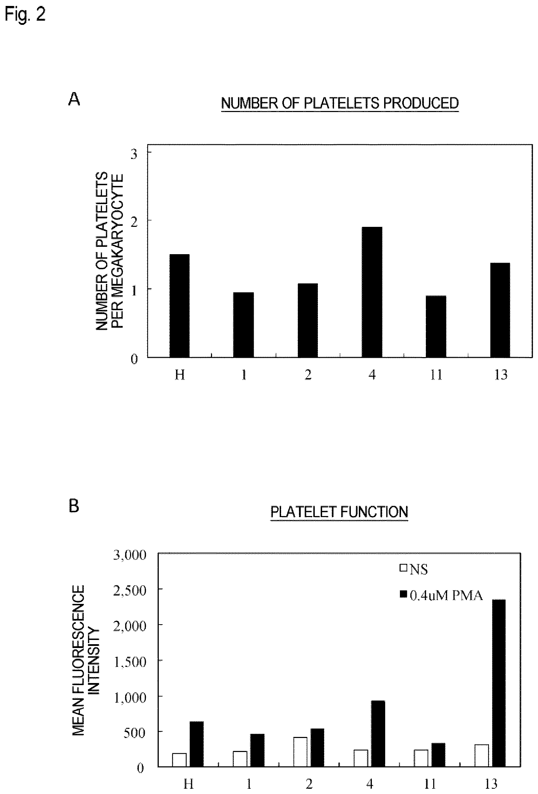

Fig. 2 is a graph indicating the results of re-measuring the number of platelets produced derived from five of the 22 megakaryocyte progenitor cell clone candidates of Fig. 1 along with megakaryocyte progenitor cells (H) composed of various original populations (Fig. 2A), and the fluorescence intensity in lieu of the amount of GPIIb/IIIa complex formed by PMA stimulation of the platelets (Fig. 2B).

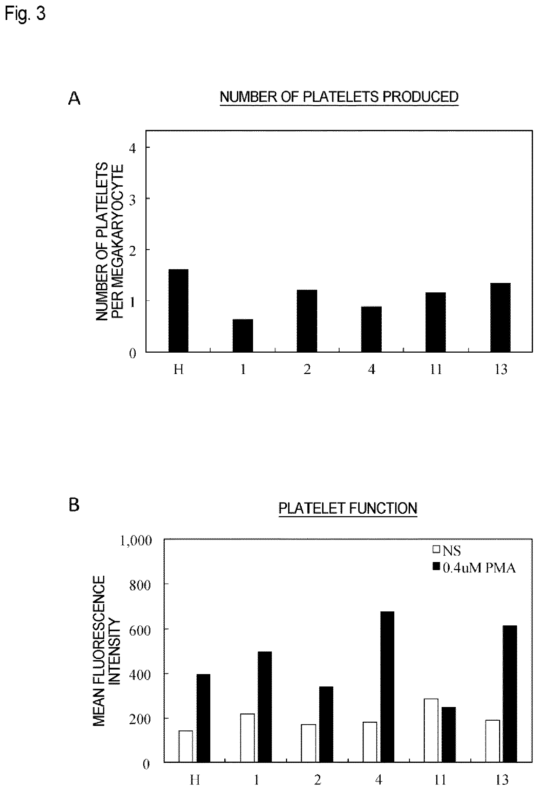

Fig. 3 is a graph indicating the results of re-measuring the numbers of platelets produced derived from the 5 megakaryocyte progenitor cell clones of Fig. 2 along with the original megakaryocyte progenitor cells (H) (Fig. 3A), and the fluorescence intensity in lieu of the amount of GPIIb/IIIa complex formed by PMA stimulation of the platelets (Fig. 3B).

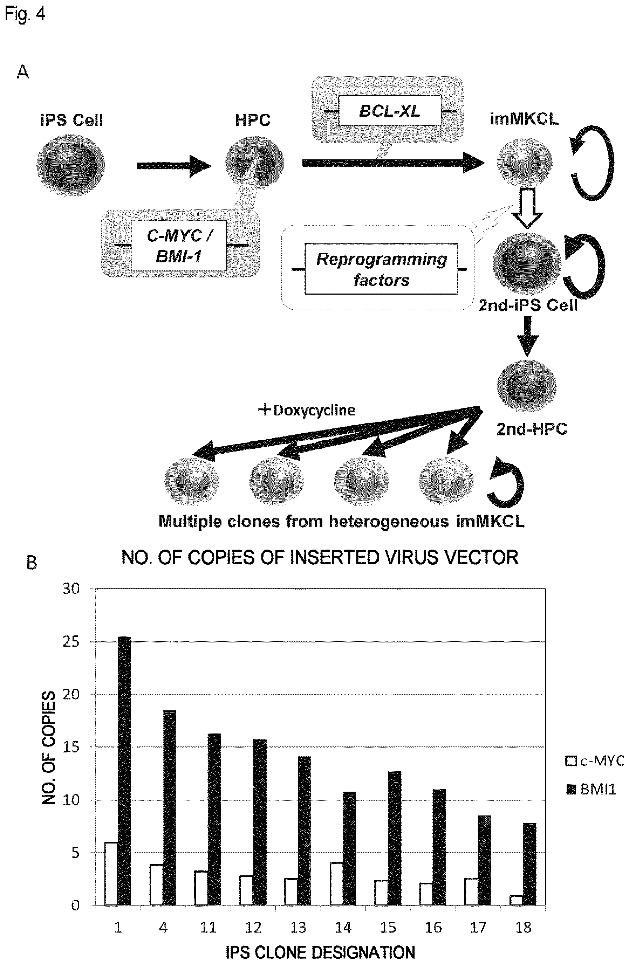

Fig. 4A is a schematic diagram of the production of megakaryocyte progenitor cells, cloning by reprogramming factors, and the production of secondary megakaryocyte progenitor cells from secondary iPS cells. Fig. 4B is a graph indicating the number of copies per c-MYC or BMI1 cell contained in the chromosomes of secondary iPS cell clones obtained by reprogramming megakaryocyte progenitor cells.

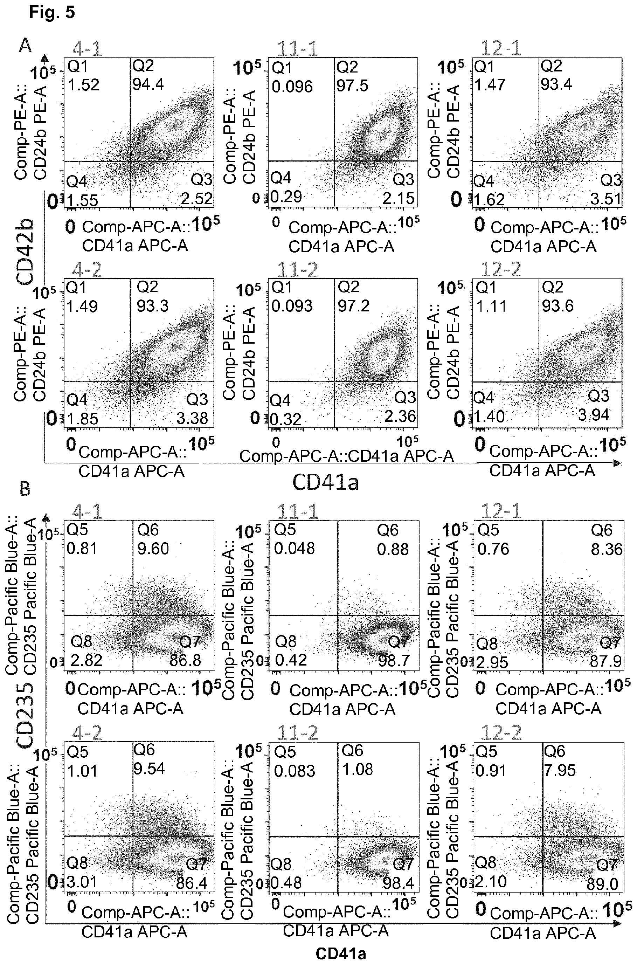

Fig. 5 shows the results of using a flow cytometer to measure the distribution of cells expressing CD42b and CD41a and the distribution of cells expressing CD235 and CD41a among megakaryocyte progenitor cells derived from each secondary iPS cell clone.

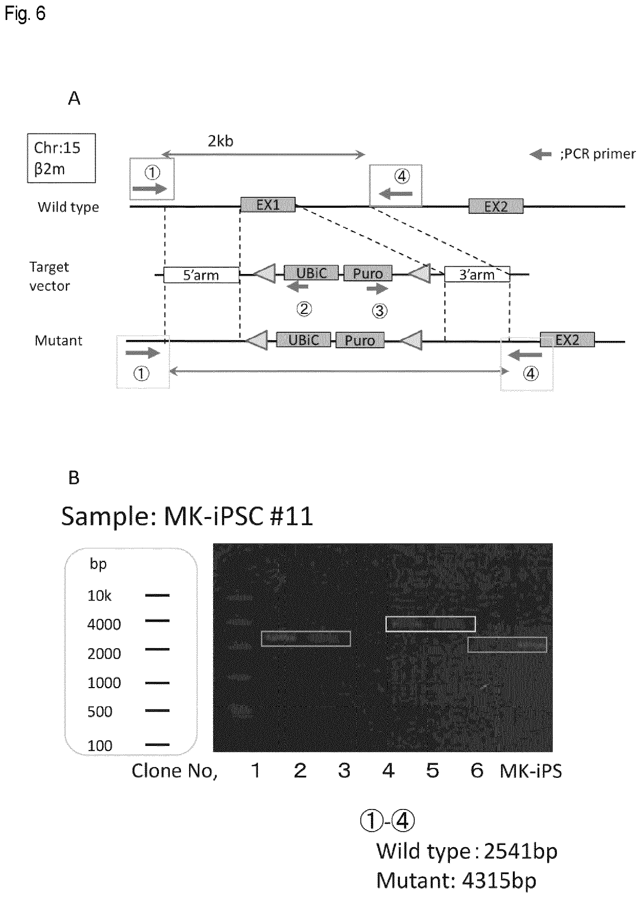

Fig. 6A is a schematic diagram of homologous recombination for deleting an Exon1 of β2-microglobulin. Fig. 6B shows the results of PCR for confirming homologous recombination in secondary iPS cell clones following introduction of a Target vector.

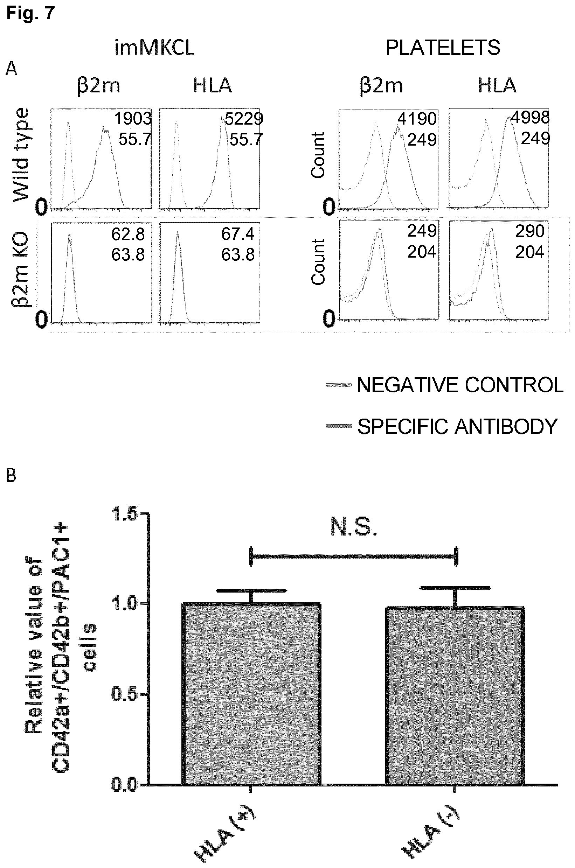

Fig. 7A indicates the results of using a flow cytometer to measure the distribution of cells expressing β2-microglobulin and HLA in megakaryocyte progenitor cells (imMKCL) derived from secondary iPS cell clones deleted of β2-microglobulin Exon1 (shown on left) and platelets produced from the megakaryocyte progenitor cells (shown on right). Fig. 7B indicates the ratio of fluorescence intensity of PAC1 following PMA stimulation in platelets produced from megakaryocytes derived from secondary iPS cell clones (HLA(-)) deleted of β2-microglobulin Exon1 or secondary iPS cell clones (HLA(+)). Fluorescence intensity is shown based on a value of 1 for platelets derived from secondary iPS cell clones (HLA(+)).

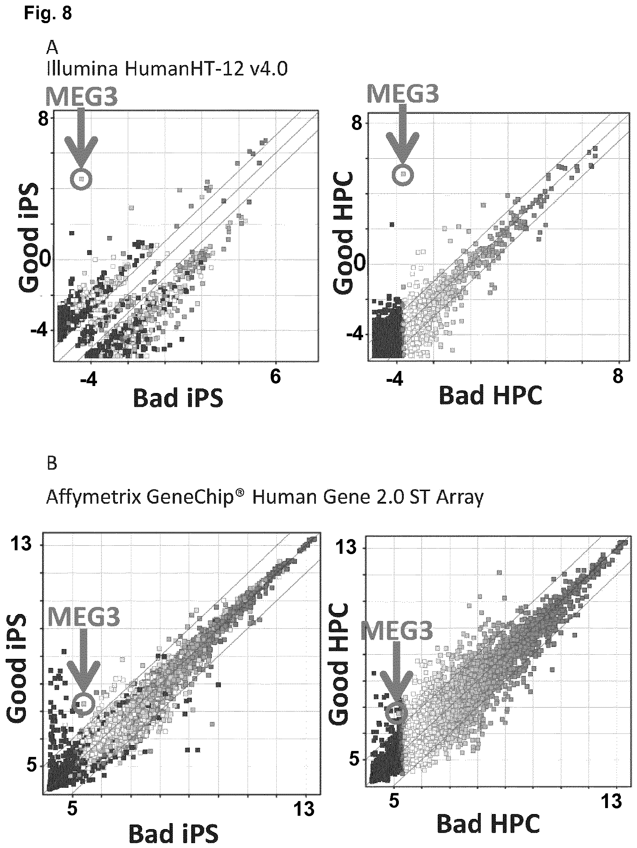

Fig. 8 indicates the results of comparing gene expression levels with a microarray available from Illumina (Fig. 8A) or Affymetrix (Fig. 8B) for secondary iPS cell clones (Good iPS) capable of being induced to differentiate into megakaryocyte progenitor cells and secondary iPS cell clones (Bad iPS) not capable of being induced to differentiate into megakaryocyte progenitor cells, or hematopoietic progenitor cells derived from secondary iPS cell clones (Good HPC) capable of being induced to differentiate into megakaryocyte progenitor cellsand secondary iPS cell clones (Bad HPC) not capable of being induced to differentiate into megakaryocyte progenitor cells.

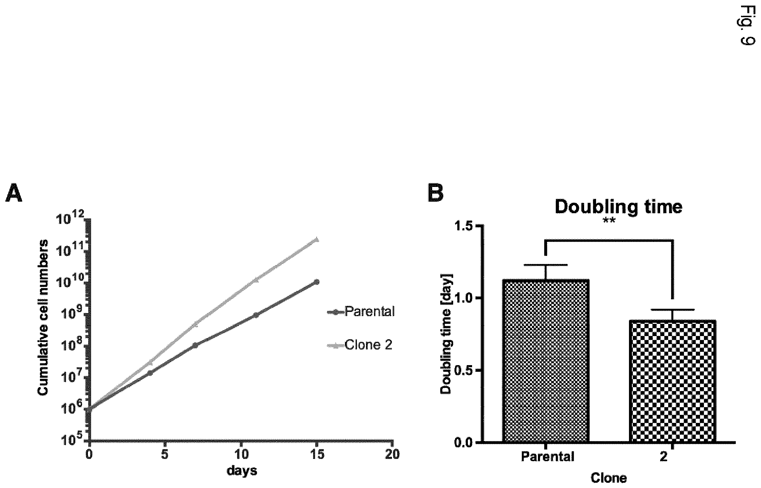

Figs. 9A and 9B respectively indicate growth curves of megakaryocytes (Clone 2) derived from a secondary iPS cell clone and megakaryocyte progenitor cells (Parental) prior to cloning, and doubling times calculated from the growth curves (**: P<0.01).

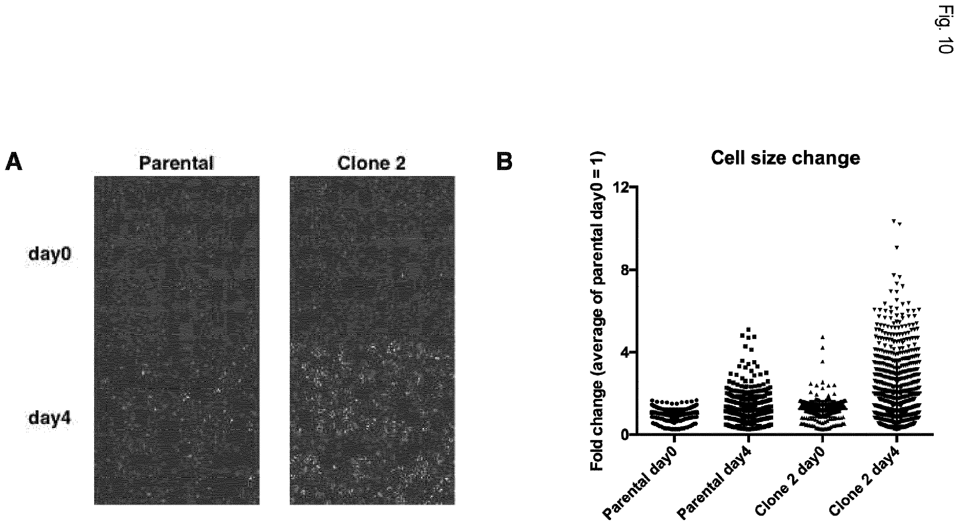

Figs. 10A and 10B respectively indicate cell appearance and changes in cell size before and after maturation of megakaryocytes (Clone 2) derived from a secondary iPS cell clone and megakaryocyte progenitor cells (Parental) prior to cloning.

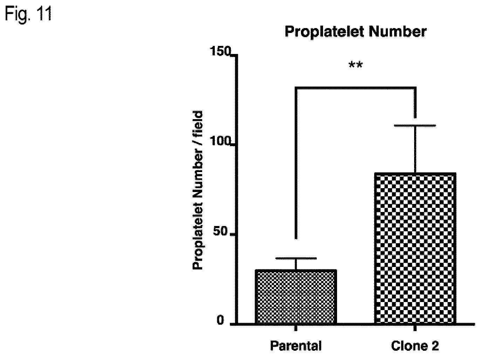

Fig. 11 is a graph comparing the numbers of proplatelets, which are progenitors of platelets, formed.

### Description of Embodiments

The method for producing a stem cell clone according to the present invention comprises the steps as defined in the claims.

Isolated stem cell clones are more suitable for being induced to differentiate into somatic cells in comparison with stem cells prior to cloning, and have a high efficiency of being induced to differentiate into somatic cells per cell.

In the present invention, cloning refers to cloning of a cell population in the sense of isolating a cell population having uniform genetic information from a cell population having non-uniform genetic information.

### Somatic Cells Submitted for Cloning

The somatic cells of the claims are megakaryocyte progenitor cells. There are no particular limitations on the somatic cells described herein submitted for cloning (to be referred to as primary somatic cells) provided they are cells produced by incorporating a gene functionally linked to a drug-responsive promoter in a chromosome therof, and examples thereof that are described herein include nerve cells (WO 2014/148646, Wapinski OL et al, Cell. 155:621-635, 2013), neural stem cells (Han DW et al, Cell Stem Cell. 10:465-472, 2012), neural crest cells (Kim YJ, et al, Cell Stem Cell. 15:497-506, 2014), myocardial cells (leda M et al, Cell. 142:375-386, 2010), skeletal muscle cells (Tanaka A, et al, PLoS One. 8:e61540, 2013), chondrocytes (Outani H, et al, PLoS One. 8:e77365, 2013), hepatocytes (Huang P, et al, Cell Stem Cell. 14:370-384, 2014), melanocytes (Yang R, et al, Nat Commun. 5:5807, 2014), hematopoietic progenitor cells (Batta K, Cell Rep. 9:1871-84, 2014), erythroblasts (Hirose S, et al, Stem Cell Reports. 1:499-508, 2013) and megakaryocyte progenitor cells (Nakamura S, et al, Cell Stem Cell. 14:535-548, 2014).

In the present invention, megakaryocyte progenitor cells are suitable as somatic cells cloned according to the method of the present invention since they cannot be cloned by the limiting dilution method.

The exogenous gene associated with induction of differentiation into somatic cells in the present invention refers in the broad sense to a gene introduced into a cell when inducing differentiation from a stem cell to a somatic cell.

The exogenous gene associated with induction of differentiation is at least one gene selected from the group consisting of oncogenes, preferably a member of the MYC gene family and more preferably c-Myc, genes suppressing expression of p16 gene or p19 gene (polycomb genes) and preferably Bmi1, and apoptosis suppressing genes and preferably BCL-XL gene.

The exogenous gene associated with induction of differentiation into somatic cells may be operably linked to a drug-responsive promoter.

In the present invention, a drug-responsive promoter refers to a promoter that expresses a gene in the presence or absence of a corresponding drug. An example of a promoter that expresses a gene in the presence of a corresponding drug is a TRE promoter (CMV minimal promoter having a Tet response sequence including seven repeats of a tet0 sequence). In the case of using a TRE promoter, a system is preferably used in which gene expression is induced in the presence of the corresponding drug (such as tetracycline or doxycycline) by simultaneously expressing a fusion protein (reverse tetR fusion protein) of reverse tetR (rtetR) and VP16AD within the same cells. In the case of using a reverse tetR fusion protein, the fusion protein is at least required to be expressed in a "step for inducing differentiation from stem cells to secondary somatic cells" to be subsequently described. For example, by functionally linking a gene encoding a reverse tetR fusion protein to a drug-responsive promoter and introducing that gene when preparing primary somatic cells, the reverse tetR fusion protein can be expressed by addition or removal of the corresponding drug in the "step for inducing differentiation from stem cells to secondary somatic cells". In the case of functionally linking a drug-responsive promoter to two or more types of genes, the same type of drug-responsive promoter may be used for all of the genes or two or more types of drug-responsive promoters may be used.

### Method for Deriving Primary Megakaryocyte Progenitor Cells

In the present invention, "megakaryocyte progenitor cells" refer to cells that become megakaryocytes as a result of maturing. These cells are not multinucleated, and include cells characterized as CD41a-positive/CD42a-positive/CD42b-weakly positive. The megakaryocyte progenitor cells of the present invention are preferably cells that can be grown by expansion culturing, such as cells capable of undergoing expansion culturing for at least 60 days under suitable conditions. In the present invention, megakaryocyte progenitor cells may or may not be cloned, and although there are no particular limitations thereon, those that have been cloned are referred to as a megakaryocyte progenitor cell line. Megakaryocyte progenitor cells in the present invention may be derived from hematopoietic progenitor cells.

In the present invention, "megakaryocytes" are also referred to as platelet progenitor cells and megakaryocytic cells, are cells that produce platelets by separation of their cytoplasm, may be multinucleated cells, and include cells characterized as, for example, CD41a-positive/CD42a-positive/CD42b-positive. In addition, megakaryocytes may also be characterized as cells expressing GATA1, FOG1, NF-E2 and β1-tubulin. Multinucleated megakaryocytes refer to a cell or group of cells in which the number of nuclei has undergone a relative increase in comparison with megakaryocyte progenitor cells. For example, in the case the nuclei of megakaryocyte progenitor cells to which the method of the present invention is applied are 2N, cells in which the nuclei thereof are 4N or more are multinucleated megakaryocytes. In addition, in the present invention, megakaryocytes may be immortalized in the form of a megakaryocyte cell line or may be a cloned cell group.

In the present invention, hematopoietic progenitor cells (HPC) refer to cells able to differentiate into blood cells such as lymphocytes, eosinophils, neutrophils, basophils, erythrocytes or megakaryocytes, and in the present invention, there is no distinction made between hematopoietic progenitor cells and hematopoietic stem cells, and refer to the same cells unless specifically indicated otherwise. Hematopoietic stem cells/progenitor cells can be recognized by, for example, being positive for the surface antigens, CD34 and/or CD43. In the present invention, hematopoietic stem cells can also be applied to hematopoietic progenitor cells that have been induced to differentiate from pluripotent stem cells or hematopoietic stem cells as well as progenitor cells derived from placental blood, bone marrow blood or peripheral blood. For example, in the case of using pluripotent stem cells, hematopoietic progenitor cells can be prepared from a net-like structure (ES-sac or iPS-sac) obtained by culturing pluripotent stem cells on C3H10T1/2 in the presence of VEGF in accordance with the method described in Takayama N., et al. J Exp Med. 2817-2830 (2010). Here, "net-like structure" refers to a three-dimensional sac-like structure (having a space inside) derived from pluripotent stem cells that is formed by an endothelial cell population or the like and contains hematopoietic progenitor cells in the interior thereof. Other examples of methods used to induce differentiation from pluripotent stem cells to hematopoietic progenitor cells include a method that uses the formation of an embryoid body and the addition of a cytokine (Chadwick et al. Blood 2003, 102: 906-15, Vijayaragavan et al. Cell Stem Cell 2009, 4: 248-62, Saeki et al. Stem Cells 2009, 27: 59-67) and a method including co-culturing with stromal cells derived from different species (Niwa A et al. J Cell Physiol. 2009 Nov;221(2):367-77.).

Examples of pluripotent stem cells include fertilized eggs and cells such as embryonic stem cells (ES cells), induced pluripotent stem cells (iPS cells) or embryonic germ cells (EG cells). Megakaryocyte progenitor cells serving as somatic cells of the present invention are preferably those that have been induced by a step for culturing the cells by overexpressing an oncogene, gene suppressing expression of p16 gene or p19 gene (polycomb gene) and/or gene suppressing apoptosis in hematopoietic progenitor cells.

In the present invention, a "oncogene" refers to a gene that causes a malignant transformation of normal cells as a result of the expression, structure or function thereof being different from that of normal cells, and examples thereof include MYC family genes, Src family genes, Ras family genes, Raf family genes, c-Kit and protein kinase family genes such as PDGFR or Abl. Examples of MYC family genes include c-MYC, N-MYC and L-MYC. c-MYC genes refer to, for example, genes composed of a nucleic acid sequence represented by NCBI accession no. NM 002467. In addition, c-MYC genes include homologs thereof, and c-MYC gene homologs refer to genes for which, for example, the cDNA sequence thereof is composed of a sequence that is substantially identical to the nucleic acid sequence represented by NCBI accession no. NM 002467. cDNA composed of a sequence substantially identical to the nucleic acid sequence represented by NCBI accession no. NM 002467 refers to DNA composed of a sequence having identify of about 60% or more, preferably about 70% or more, more preferably about 80%, 81%, 82%, 83%, 84%, 85%, 86%, 87%, 88%, 89%, 90%, 91%, 92%, 93%, 94%, 95%, 96%, 97% or 98%, and most preferably about 99%, with DNA composed of a sequence represented by NCBI accession no. NM 002467, or DNA able to hybridize under stringent conditions with DNA composed of a sequence complementary to the nucleic acid sequence represented by NCBI accession no. NM 002467, with protein encoded by these DNA contributing to expansion of cells such as hematopoietic progenitor cells that are in a stage of differentiation.

Here, stringent conditions refer to hybridization conditions easily determined by a person with ordinary skill in the art that are typically empirical experimental conditions dependent on probe length, washing temperature and salt concentration. In general, the temperature for proper annealing becomes higher as probe length increases, and the temperature becomes lower as probe length decreases. Hybrid formation is typically dependent on the ability of the complementary strand to undergo repeat annealing in an environment at a temperature somewhat lower than the melting point thereof.

For example, an example of lowly stringent conditions includes washing at 0.1 × SSC in a 0.1% SDS solution under temperature conditions of 37°C to 42°C during the stage of washing the filter following hybridization. In addition, an example of highly stringent conditions includes washing at 5 × SSC in a 0.1% SDS solution at 65°C during the washing stage. Polynucleotides of higher homology can be obtained by using more highly stringent conditions.

In the present invention, since it is preferable to suppress the expression level of c-MYC, the c-MYC may be that which encodes a protein fused with a destabilizing domain. A destabilizing domain acquired from ProteoTuner or Clontech Laboratories, Inc. can be used.

In the present invention, examples of "genes suppressing expression of p16 gene or p19 gene" include BMI1, Id1, Mel18, Ring1a/b, Phc1/2/3, Cbx2/4/6/7/8, Ezh2, Eed, Suz12, HDAC and Dnmt1/3a/3b. BMI1 gene refers to, for example, a gene composed of a nucleic acid sequence represented by NCBI accession no. NM 005180. In addition, BMI1 gene includes homologs thereof, and homologs of BMI1 gene refer to genes for which the cDNA sequence thereof is composed of a sequence substantially identical to the nucleic acid sequence represented by NCBI accession no. NM 005180. cDNA composed of a sequence substantially identical to the nucleic acid sequence represented by NCBI accession no. NM 005180 refers to refers to DNA composed of a sequence having identify of about 60% or more, preferably about 70% or more, more preferably about 80%, 81%, 82%, 83%, 84%, 85%, 86%, 87%, 88%, 89%, 90%, 91%, 92%, 93%, 94%, 95%, 96%, 97% or 98%, and most preferably about 99%, with DNA composed of the sequence represented by NCBI accession no. NM 005180, or DNA able to hybridize under stringent conditions with DNA composed of a sequence complementary to the nucleic acid sequence represented by NCBI accession no. NM 005180, with protein encoded by that DNA promoting cell expansion by suppressing senescence of cells capable of inducing oncogenes occurring in cells that are expressed by oncogenes such as MYC family genes.

In the present invention, "apoptosis suppressing genes" refer to genes that suppress apoptosis, there are no particular limitations thereon, and examples thereof include BCL2 gene, BCL-XL gene, Survivin and MCL1. BCL-XL gene refers to a gene composed of the nucleic acid sequence represented by NCBI accession no. NM 001191 or NM 138578. In addition, BCL-XL gene includes homologs thereof, and BCL-XL gene homologs refer to genes for which, for example, the cDNA sequence thereof is composed of a sequence that is substantially identical to the nucleic acid sequence represented by NCBI accession no. NM 001191 or NM 138578. cDNA composed of a sequence substantially identical to the nucleic acid sequence represented by NCBI accession no. NM 001191 or NM 138578 refers to DNA composed of a sequence having identify of about 60% or more, preferably about 70% or more, more preferably about 80%, 81%, 82%, 83%, 84%, 85%, 86%, 87%, 88%, 89%, 90%, 91%, 92%, 93%, 94%, 95%, 96%, 97% or 98%, and most preferably about 99%, with DNA composed of a sequence represented by NCBI accession no. NM 001191 or NM 138578, or DNA able to hybridize under stringent conditions with DNA composed of a sequence complementary to the nucleic acid sequence represented by NCBI accession no. NM 001191 or NM 138578, with protein encoded by this DNA having the effect of suppressing apoptosis.

In the present invention, the method for overexpressing the above-mentioned genes in hematopoietic progenitor cells is preferably carried out by incorporating a gene functionally linked to a drug-responsive promoter in a chromosome thereof, and can be achieved by, for example, introducing an expression vector containing a gene functionally linked to a drug-responsive promoter into a hematopoietic progenitor cell. Examples of vectors expressing these genes that can be used include retrovirus, lentivirus and other virus vectors as well as animal cell expression plasmids (such as pA1-11, pXT1, pRc/CMV, pRc/RSV or pcDNAI/Neo). Retrovirus vectors or lentivirus vectors are used preferably from the viewpoint of incorporating in a chromosome.

In addition to a promoter, the expression vector may contain an enhancer, Poly(A) addition signal, selection marker gene or SV40 replication origin and the like. Examples of useful selection marker genes include dihydrofolate reductase gene, neomycin resistance gene and puromycin resistance gene.

In the present invention, a polycistronic vector, in which genes are linked longitudinally, may be obtained in order to introduce a plurality of genes simultaneously. In order to enable polycistronic expression, the 2A self-cleaving peptide of foot and mouth disease virus (refer to, for example, Science, 322, 949-953, 2008), or an IRES (Internal ribosome entry site) sequence, may be ligated between a plurality of overexpressed genes.

In the present invention, in the case of a virus vector, the method for introducing an expression vector into hematopoietic progenitor cells can be carried out by introducing a plasmid containing the nucleic acid into suitable packaging cells (such as Plat-E cells) or a complementing cell line (such as 293 cells) followed by recovering virus produced in the culture supernatant and infecting hematopoietic progenitor cells by contacting with the virus. In the case of a non-virus vector, a plasmid vector can be introduced into cells by using a method such as lipofection, liposome method, electroporation, calcium phosphate co-precipitation, DEAE dextran method, microinjection or a gene gun.

In one aspect of the method for inducing megakaryocyte progenitor cells according to the present invention, apoptosis suppressing gene may be overexpressed after having overexpressed an oncogene or gene suppressing expression of p16 gene or p19 gene in hematopoietic progenitor cells. Overexpression of apoptosis suppressing gene can be carried out in the same manner as described above by introducing an expression vector, protein encoded by these genes, or RNA encoding these genes, into hematopoietic progenitor cells. In the case of subsequent expression of apoptosis suppressing gene, although there are no particular limitations thereon, overexpression of apoptosis suppressing gene is preferably carried out after overexpressing an oncogene or gene suppressing expression of p16 gene or p19 gene for at least 14 days.

In the present invention, a caspase inhibitor may be contacted with the hematopoietic progenitor cells instead of overexpressing apoptosis suppressing gene in the cells. In the present invention, the caspase inhibitor may be any of a peptidic compound, non-peptidic compound or biological protein. Examples of peptidic compounds include the artificial chemically synthesized peptidic compounds indicated in (1) to (10) below.
(1) Z-Asp-CH2-DCB (molecular weight: 454.26)
(2) Boc-Asp(OMe)-FMK (molecular weight: 263.3)
(3) Boc-Asp(OBzl)-CMK (molecular weight: 355.8)
(4) Ac-AAVALLPAVLLALLAP-YVAD-CHO (molecular weight: 1990.5) (SEQ ID NO: 1)
(5) Ac-AAVALLPAVLLALLAP-DEVD-CHO (molecular weight: 2000.4) (SEQ ID NO: 2)
(6) Ac-AAVALLPAVLLALLAP-LEVD-CHO (molecular weight: 1998.5) (SEQ ID NO: 3)
(7) Ac-AAVALLPAVLLALLAP-IETD-CHO (molecular weight: 2000.5) (SEQ ID NO: 4)
(8) Ac-AAVALLPAVLLALLAP-LEHD-CHO (molecular weight: 2036.5) (SEQ ID NO: 5)
(9) Z-DEVD-FMK (Z-Asp-Glu-Val-Asp-fluoromethylketone) (SEQ ID NO: 6)
(10) Z-VAD FMK

Examples of caspase inhibitors of peptidic compounds include: (1) VX-740 - Vertex Pharmaceuticals (Leung-Toung et al., Curr. Med. Chem. 9, 979-1002 (2002)) and (2) HMR-3480 - Aventis Pharma AG (Randle et al., Expert Opin. Investig. Drugs 10, 1207-1209 (2001)).

Examples of caspase inhibitors of non-peptidic compounds include: (1) Anilinoquinazolines (AQZs), AstraZeneca Pharmaceuticals (Scott et al., J. Pharmacol. Exp. Ther. 304, 433-440 (2003)), (2) M826 - Merck Frosst (Han et al., J. Biol. Chem. 277, 30128-30136 (2002)), (3) M867 - Merck Frosst (Methot et al., J. Exp. Med. 199, 199-207 (2004)), and (4) Nicotinyl aspartyl ketones - Merck Frosst (Isabel et al., Bioorg. Med. Chem. Lett. 13, 2137-2140 (2003)).

In addition, examples of caspase inhibitors of other non-peptidic compounds include: (1) IDN-6556 - Idun Pharmaceuticals (Hoglen et al., J. Pharmacol. Exp. Ther. 309, 634-640 (2004)), (2) MF-286 and MF-867 - Merck Frosst (Los et al., Drug Discov. Today 8, 67-77 (2003)), (3) IDN-5370 - Idun Pharmaceuticals (Deckwerth et al., Drug Dev. Res. 52, 579-586 (2001)), (4) IDN-1965 - Idun Pharmaceuticals (Hoglen et al., J. Pharmacol. Exp. Ther. 297, 811-818 (2001)), and (5) VX-799 - Vertex Pharmaceuticals (Los et al., Drug Discov. Today 8, 67-77 (2003)). Other examples of caspase inhibitors include M-920 and M-791 - Merck Frosst (Hotchkiss et al., Nat. Immunol. 1, 496-501 (2000)).

In the present invention, the caspase inhibitor is preferably Z-VAD FMK. In the case of using Z-VAD FMK for the caspase inhibitor, the Z-VAD FMK is added to the medium in which hematopoietic progenitor cells are cultured. The preferable concentration of Z-VAD FMK in the medium is, for example, 10 µM or more, 20 µM or more, 30 µM or more, 40 µM or more or 50 µM or more, and is preferably 30 µM or more.

Although there are no particular limitations thereon, the medium used to derive megakaryocyte progenitor cells from hematopoietic progenitor cells can be prepared by using medium used to culture animal cells as basal medium. Examples of basal media include IMDM medium, Medium 199 medium, Eagle's Minimum Essential Medium (EMEM), αMEM medium, Dulbecco's modified Eagle's Medium (DMEM), Ham's F12 medium, RPMI 1640 medium, Fischer's medium, Neurobasal Medium (Life Technologies) and mixed media thereof. The medium may contain serum or may be serum-free. The medium can also contain one or more substances such as albumin, insulin, transferrin, selenium, fatty acids, trace elements, 2-mercaptoethanol, thiol glycerol, lipid, amino acids, L-glutamine, non-essential amino acids, vitamins, growth factors, low molecular weight compounds, antibiotics, antioxidants, pyruvic acid, buffers, inorganic salts or cytokines as necessary. Cytokines refer to proteins that promote hematopoietic differentiation, and examples thereof include VEGF, TPO and SCF. Preferable medium in the present invention is IMDM medium containing serum, insulin, transferrin, serine, thiol glycerol, ascorbic acid and TPO. The medium more preferably further contains SCF. In addition, in the case of using an expression vector containing a drug-responsive promoter, a corresponding drug such as tetracycline or doxycycline is preferably contained in the medium in the overexpression step.

In the present invention, although there are no particular limitations thereon, temperature conditions for deriving megakaryocyte progenitor cells from hematopoietic progenitor cells are such that promotion of differentiation into megakaryocyte progenitor cells is confirmed by culturing hematopoietic progenitor cells at a temperature of 37°C or higher. Here, since a temperature that does not impart damage to cells is suitable, a temperature of 37°C or higher refers to, for example, a temperature of about 37°C to about 42°C and preferably a temperature of about 37°C to about 39°C. The duration of culturing at a temperature of 37°C or higher can be suitably determined by a person with ordinary skill in the art while monitoring such factors as the number of megakaryocyte progenitor cells. Although there are no particular limitations on this duration provided a desired number of megakaryocyte progenitor cells are obtained, examples thereof include a duration of at least 6 days or more, 12 days or more, 18 days or more, 24 days or more, 30 days or more, 42 days or more, 48 days or more, 54 days or more or 60 days or more, and preferably 60 days or more. A long culturing period does not present a problem with respect to induction of megakaryocyte progenitor cells. In addition, subculturing is preferably suitably carried out during the culturing period.

### Method for Reprogramming Somatic Cells

In the present invention, the introduction of a reprogramming factor as claimed into somatic cells can be carried out for the method used to reprogram somatic cells. Reprogramming factors described herein include genes or gene products such as Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3 or Glis1, and these reprogramming factors may be used alone or in combination. Combinations of reprogramming factors described herein include the combinations described in WO 2007/069666, WO 2008/118820, WO 2009/007852, WO 2009/032194, WO 2009/058413, WO 2009/057831, WO 2009/075119, WO 2009/079007, WO 2009/091659, WO 2009/101084, WO 2009/101407, WO 2009/102983, WO 2009/114949, WO 2009/117439, WO 2009/126250, WO 2009/126251, WO 2009/126655, WO 2009/157593, WO 2010/009015, WO 2010/033906, WO 2010/033920, WO 2010/042800, WO 2010/050626, WO 2010/056831, WO 2010/068955, WO 2010/098419, WO 2010/102267, WO 2010/111409, WO 2010/111422, WO 2010/115050, WO 2010/124290, WO 2010/147395, WO 2010/147612, Huangfu D, et al. (2008), Nat. Biotechnol., 26: 795-797, Shi Y, et al. (2008), Cell Stem Cell, 2: 525-528, Eminli S, et al. (2008), Stem Cells. 26:2467-2474, Huangfu D, et al. (2008), Nat. Biotechnol. 26:1269-1275, Shi Y, et al. (2008), Cell Stem Cell, 3, 568-574, Zhao Y, et al. (2008), Cell Stem Cell, 3:475-479, Marson A, (2008), Cell Stem Cell, 3, 132-135, Feng B, et al. (2009), Nat. Cell Biol. 11:197-203, R. L. Judson et al., (2009), Nat. Biotechnol., 27:459-461, Lyssiotis CA, et al. (2009), Proc Natl Acad Sci USA. 106:8912-8917, Kim JB, et al. (2009), Nature. 461:649-643, Ichida JK, et al. (2009), Cell Stem Cell. 5:491-503, Heng JC, et al. (2010), Cell Stem Cell. 6:167-74, Han J, et al. (2010), Nature. 463:1096-100, Mali P, et al. (2010), Stem Cells. 28:713-720 and Maekawa M, et al. (2011), Nature. 474:225-9. A more preferable combination of reprogramming factors includes Oct3/4, Sox2 and Klf4.

The above-mentioned reprogramming factors contain factors used for the purpose of enhancing establishment efficiency such as histone deacetylase (HDAC) inhibitors (such as small molecule inhibitors in the manner of valproic acid (VPA), trichostatin A, sodium butyrate, MC 1293 or M344, nucleic acid expression inhibitors such as siRNA and shRNA against HDAC (for example, HDAC1 siRNA Smartpool^{®} (Millipore), HuSH 29mer shRNA Constructs against HDAC1 (Ori Gene)), MEK inhibitors (such as PD184352, PD98059, U0126, SL327 or PD0325901), glycogen synthase kinase-3 inhibitors (such as Bio or CHIR99021), DNA methyl transferase inhibitors (such as 5-azacytidine), histone methyl transferase inhibitors (such as small molecule inhibitors in the manner of BIX-01294 or nucleic acid expression inhibitors in the manner of siRNA and shRNA against Suv39hl, Suv39h2, SetDBI or G9a), L-channel calcium agonists (such as Bayk8644), butyric acid, TGFβ inhibitors or ALK5 inhibitors (such as LY364947, SB431542, 616453 orA-83-01), p53 inhibitors (such as siRNA and shRNA against p53), ARID3A inhibitors (such as siRNA and shRNA against ARID3A), miRNA such as miR-291-3p, miR-294, miR-295 or miR-302), Wnt signaling (such as soluble Wnt3a), neuropeptide Y, prostaglandins (such as prostaglandin E2 or prostaglandin J2), hTERT, SV40LT, UTF1, IRX6, GLISI, PITX2 or DMRTBI, and in the present description, there are no particular distinctions made reprogramming factors and these factors used for the purpose of improving establishment efficiency.

In the case the reprogramming factor is in the form of a protein, the reprogramming factor may be introduced into somatic cells by a technique such as lipofection, fusion with a cell-permeating peptide (such as HIV-derived TAT or polyarginine), or microinjection.

On the other hand, in the case the reprogramming factor is in the form of DNA, DNA can be introduced into somatic cells by a vector in the manner of a virus, plasmid or artificial chromosome, and by means such as ipofection, liposomes or microinjection. Examples of virus vectors include retrovirus vector, lentivirus vector (described in Cell, 126, pp. 663-676, 2006; Cell, 131, pp. 861-872, 2007; Science, 318, pp. 1917-1920, 2007), adenovirus vector (Science, 322, 945-949, 2008), adeno-associated virus vector, and Sendai virus vector (WO 2010/008054). In addition, examples of artificial chromosome vectors include human artificial chromosomes (HAC), yeast artificial chromosomes (YAC) and bacterial artificial chromosomes (BAC, PAC). Mammalian cell plasmids can be used as plasmids (Science, 322:949-953, 2008). Vectors can contain a control sequence such as a promoter, enhancer, ribosome binding sequence, terminator or polyadenylation site to enable expression of nuclear reprogramming substance, and can further contain a drug resistance gene (such as kanamycin resistance gene, ampicillin resistance gene or puromycin resistance gene), selection marker sequence such as thymidine kinase gene or diphtheria toxin gene, or a reporter sequence gene such as green fluorescent protein (GFP), β-glucuronidase (GUS) or FLAG as necessary. In addition, the above-mentioned vectors may have an LoxP sequence before or after the vector in order to remove genes encoding the reprogramming factors or both a promoter and gene encoding a reprogramming factor bound thereto, following introduction into somatic cells.

In the case the reprogramming factor is in the form of RNA, the reprogramming factor can be introduced into somatic cells by a technique such as lipofection or microinjection, and RNA incorporating 5-methylcytidine and pseudouridine (TriLink Biotechnologies) may be used to inhibit degradation (Warren L, (2010) Cell Stem Cell. 7:618-630).

Examples of culture broth for cells following reprogramming include DMEM containing 10% to 15% FBS, DMEM/F12 and DME culture broth (and these culture broths can suitably further contain LIF, penicillin/streptomycin, puromycin, L-glutamine, non-essential amino acids or β-mercaptoethanol and the like), as well as commercially available culture broths (such as culture broth for culturing mouse ES cells (TX-WES culture broth, Thrombo-X), culture broth for culturing primate ES cells (Primate ES/iPS cell culture broth, ReproCELL Inc.), or serum-free medium (mTeSR, Stemcell Technology)).

An example of a method for culturing cells following reprogramming comprises contacting somatic cells with reprogramming factor in DMEM containing 10% FBS or DMEM/F12 culture broth at 37°C in the presence of 5% CO₂ and culturing for about 4 days to 7 days, followed by reseeding the cells in feeder cells (such as mitomycin C-treated STO cells or SNL cells), and culturing in culture broth for culturing primate ES cells containing bFGF starting about 10 days after contacting the somatic cells with the reprograming factor to allow the formation of iPS-like colonies after about 30 days to about 45 days or more from the time of contact.

Alternatively, somatic cells are cultured in DMEM medium containing 10% FBS (which may also suitably contain LIF, penicillin/streptomycin, puromycin, L-glutamine, non-essential amino acids or β-mercaptoethanol and the like) in feeder cells (such as mitomycin C-treated STO cells or SNL cells) at 37°C in the presence of 5% CO₂ to allow the formation of ES-like colonies after about 25 days to about 30 days. The reprogrammed somatic cells are preferably used as is instead of feeder cells (Takahashi K, et al. (2009), PLoS One. 4:e8067 or WO 2010/137746), or an extracellular matrix is used (such as Laminin-5 (WO 2009/123349) or Matrigel (Becton, Dickinson and Company)).

In addition, examples of culturing methods include methods using medium that does not contain serum (Sun N, et al. Proc Natl Acad Sci USA. 106:15720-15725, 2009 or Nakagawa M, et al, Sci Rep. 4:3594, 2014). Moreover, iPS cells may be established under hypoxic conditions (oxygen concentration of 0.1% to 15%) in order to increase establishment efficiency (Yoshida Y, et al. (2009), Cell Stem Cell. 5:237-241 or WO 2010/013845).

The culture broth is replaced with fresh culture broth once a day starting on day 2 after the start of culturing during the above-mentioned culturing. In addition, although there are no particular limitations thereon, an example of the number of somatic cells used in reprogramming is within the range of about 5 × 10³ to about 5 × 10⁶ cells per 10 cm² of culture dish area.

### Step for Isolating Stem Cell Colonies Obtained by Reprogramming Somatic Cells

In the present invention, stem cell colonies can be obtained by introducing a reprogramming factor into somatic cells and culturing the cells as described above. In the present invention, stem cells refer to cells having a self-replication ability, which enables the cells to produce cells identical to those cells by cell division, and an ability to differentiate into different types of cells, while also being able to proliferate without limitation. Although there are no particular limitations on the stem cells of the present invention provided they form colonies, these stem cells are pluripotent stem cells having the ability to differentiate to tissue cells excluding placental cells.

In the present invention, a colony refers to a cell mass derived from a single cell.

The cloning method of the present invention comprises a step for isolating the resulting stem cell colonies. This isolation can be carried out by suitably harvesting a single colony and then transferring to another culture dish.

### iPS Cells for Inducing Megakaryocyte Progenitor Cells

In the present invention, in the case the somatic cells are megakaryocyte progenitor cells, an exogenous oncogene and an exogenous gene suppressing expression of p16 gene or p19 gene functionally linked to a drug-responsive promoter may be contained in the chromosomes of megakaryocyte progenitor cells as previously described. In this case, secondary iPS cells, obtained by reprogramming the megakaryocyte progenitor cells according to the method described above, similarly contain an exogenous oncogene and an exogenous gene suppressing expression of p16 gene or p19 gene functionally linked to a drug-responsive promoter in the chromosomes thereof. At this time, the content ratio of exogenous gene suppressing expression of p16 gene or p19 gene functionally linked to a drug-responsive promoter to the exogenous oncogene functionally linked to a drug-responsive promoter in the iPS cells for inducing megakaryocyte progenitor cells is preferably 2-fold to 7-fold and more preferably 3-fold to 5-fold. Similarly, the content ratio of exogenous gene suppressing expression of p16 gene or p19 gene functionally linked to a drug-responsive promotor to exogenous oncogene functionally linked to a drug-responsive promoter in the megakaryocyte progenitor cells is preferably 2-fold to 7-fold and more preferably 3-fold to 5-fold. Furthermore, an oncogene and a gene that suppresses expression of p16 gene or p19 gene are suitably selected for the above-mentioned genes.

### Step for Inducing Differentiation from Stem Cells to Secondary Somatic Cells

The cloning method of the present invention comprises a step for inducing differentiation of stem cells obtained according to the method described above to secondary somatic cells. In the present invention, secondary somatic cells refer to somatic cells obtained by reprogramming primary somatic cells to stem cells followed by inducing to differentiate into secondary somatic cells, and the primary somatic cells and secondary somatic cells are preferably the same cells. The present induction step can be carried out by re-expressing an incorporated gene. Gene re-expression can be carried out by contacting cells at any stage of differentiation from stem cells, obtained by reprogramming primary somatic cells, to secondary somatic cells with a corresponding drug (in the case of a promoter that expresses a gene in the presence of a corresponding drug), or by interrupting contact between cells at any stage of differentiation from stem cells, obtained by reprogramming primary somatic cells, into secondary somatic cells and a corresponding drug (in the case of a promoter that expresses a gene when a corresponding drug is removed). For example, in the case of using a fusion gene (reverse tetR) of rtetR and VP16AD, the gene can be re-expressed by administering a corresponding drug. "Cells at any stage of differentiation from stem cells, obtained by reprogramming primary somatic cells, to secondary somatic cells" can be any cells in which the stem cells have gone through differentiation into secondary somatic cells. Thus, in the present invention, the stem cells may be induced to differentiate into other cells prior to gene re-expression, and examples of cells following this induction of differentiation include fibroblasts and hematopoietic progenitor cells. Induction of differentiation into "other cells" can be carried out in accordance with known methods. In the case of using megakaryocyte progenitor cells as somatic cells, the previously described method for inducing differentiation into hematopoietic progenitor cells is an example of a method used to induce differentiation. Namely, by administering a drug corresponding to a medium used to induce differentiation from stem cells to hematopoietic progenitor cells and induce differentiation from the previously described hematopoietic progenitor cells to megakaryocyte progenitor cells, an oncogene, gene suppressing expression of p16 gene or p19 gene, and/or apoptosis suppressing gene can be overexpressed.

### Step for Selecting Stem Cells or Hematopoietic Progenitor Cells

In the present invention, since all stem cells are not necessarily able to be induced to differentiate to megakaryocyte progenitor cells in the case of using megakaryocyte progenitor cells as somatic cells, stem cells capable of being induced to differentiate into megakaryocyte progenitor cells are selected, and an example of a method for carrying this out comprises selecting those stem cells that express MEG3. In the present invention, hematopoietic progenitor cells derived from stem cells expressing MEG3 may also be selected. In the present invention, in the case of humans, MEG3 refers to non-coding RNA composed of a nucleic acid sequence represented by NCBI accession no. NR 002766, NR 003530, NR 003531, NR 033358, NR 033359, NR 033360, NR 046464, NR 046465, NR 046466, NR 046467, NR 046468, NR 046469, NR 046470, NR 046471, NR 046472 or NR 046473. Although there are no particular limitations thereon, stem cells to which the method of the present invention is applied may be primary pluripotent stem cells and are more preferably stem cell clones obtained according to the method described above. A high level of expression may refer to expression at level that is higher than the average value in a plurality of simultaneously measured stem cells or hematopoietic progenitor cells, or may refer to an expression level that is higher in comparison with expression of known stem cells or hematopoietic progenitor cells that cannot be induced to differentiate to megakaryocyte progenitor cells.

In the present invention, a method known among persons with ordinary skill in the art can be used as a method for confirming expression of MEG3, and examples thereof include reverse transcriptase PCR analysis, quantitative reverse transcriptase PCR analysis, Northern blotting analysis, immunohistochemical analysis, array analysis and combinations thereof.

### Method for Producing Platelets

The method for producing platelets of the present claims comprises a step for cloning megakaryocyte progenitor cells using the cloning method of the present invention, and a step for allowing the cloned megakaryocyte progenitor cells to mature into megakaryocytes and release platelets. The step for allowing the cloned megakaryocyte progenitor cells to mature and release platelets can be carried out in accordance with a known method or method complying therewith. For example, in the case the megakaryocyte progenitor cells contain at least one gene selected from the group consisting of MYC family genes, polycomb genes and apoptosis suppressing genes, maturation of megakaryocytes can be carried out by suppressing expression of MYC family genes, polycomb genes and/or apoptosis suppressing genes by removing a corresponding drug from the medium following the above-mentioned step (iii). The matured megakaryocytes become multinucleated and release platelets.

The platelets may be in the form of a platelet preparation by combining with ACD-A solution, FFP, sodium citrate, citric acid or glucose and the like, or may be in the form of a blood preparation by combining with erythrocytes.

In the case of obtaining megakaryocyte clones deficient in HLA according to the method described above, platelets deficient in HLA can be obtained by allowing the megakaryocyte clones to mature and release platelets. HLA-deficient platelets are useful since they can be transfused irrespective of the HLA type of the recipient.

### Examples

### Production of Megakaryocyte Progenitor Cells

Hematopoietic progenitor cells (HPC) were derived through iPS-sac from iPS cells (SeV2: prepared by introducing c-MYC, OCT3/4, SOX2 and KLF4 into neonate human fibroblasts using a Sendai virus vector in accordance with the method described in WO 2010/134526) in a semi-confluent state and maintained in a 6 cm dish in which MEF were disseminated at 3 × 10⁵ cells/dish. More specifically, the iPS cells were separated using human trypsin solution, and about 1/30 to 1/50 of the cells were disseminated on C3H10T1/2 (available from Riken, Japan.) treated with mitomycin C (MMC) in the form of a colony mass. Furthermore, the MMC-treated C3H10T1/2 was prepared by disseminating in a 10 cm dish at 8 × 10⁵ cells/dish on the day before disseminating the iPS cells. Following dissemination, culturing was started in Eagle's Basal Medium (EBM) containing 20 ng/ml VEGF in an atmosphere of 5% O₂ and 5% CO₂ at 37°C (day 0). The medium was replaced with the same medium on day 3 and day 6.

On day 7, culturing was continued in an atmosphere of 20% O₂ and 5% CO₂ at 37°C. The medium was replaced with the same medium on day 9, day 11 and day 13. On day 14, the cells were physically detached using a cell scraper or the tip of a pipette, and cells of uniform size were recovered by passing through a 40 micrometer cell strainer. The recovered cells were confirmed to be hematopoietic progenitor cells (HPC) based on cell size.

On day 14, the recovered HPC were disseminated in MMC-processed C3H10T1/2 at 3 × 10⁴ to 1 × 10⁵ cells/well. EBM containing SCF at 50 ng/ml, TPO at 50 ng/ml and doxycycline at 0.5 µg/ml was used for the medium. Continuing, c-MYC and BMI1 were introduced into the HPC with a lentivirus vector. The lentivirus vector used was a tetracycline-controlled inducible vector, and was prepared by recombining an mOKS cassette of LV-TRE-mOKS-Ubc-tTA-I2G to c-MYC or BMI1 (LV-TRE-c-MYC-xL-Ubc-tTA-I2G or LV-TRE-BMI1-Ubc-tTA-I2G, respectively) (Nakamura S, et al, Cell Stem Cell. 14:535-548, 2014). The virus particles used for infection were prepared by infecting 293T cells with the lentivirus vector (MOI 300). Protamine was added only during infection. Subsequently, the medium was replaced every other day and the C3H10T1/2 and medium were replaced once or twice a week.

BCL-xl was introduced at MOI 10 using a lentivirus vector two weeks after introducing c-MYC and BMI1. The lentivirus vector used to introduce BCL-xl was a tetracycline-controlled inducible vector, and was prepared by recombining an mOKS cassette to contain BCL-xl in the same manner as described above (LV-TRE-BCL-xL-Ubc-tTA-I2G) (Nakamura S, et al, Cell Stem Cell. 14:535-548, 2014). Protamine was added only during infection. Subsequently, culturing was maintained in EBM containing SCF at 50 ng/ml, TPO at 50 ng/ml and doxycycline at 0.5 µg/ml in 10T1/2 feeder cells in a 10 cm dish to prepare megakaryocyte progenitor cells (to also be referred to as imMKCL).

### Reference Example 1

### Limiting Dilution Method

Megakaryocyte progenitor cells (imMKCL) were disseminated in a 96-well plate at a density of 1.5 cells/300 µL/well followed by culturing for 10 days to 14 days in Iscove's modified Dulbecco's medium (IMDM) containing 15% fetal bovine serum (FBS), human SCF (R&D Systems) at 50 ng/ml, TPO at 50 ng/ml, doxycycline (Clontech) at 5 mg/ml and puromycin (Sigma-Aldrich) at 2 mg/ml in an atmosphere of 5% CO₂ at 37°C. Culturing was continued in the same manner after transferring the contents of each well to a 24-well plate and 6-well plate for the purpose of scaling up culturing. The cells in each well were designated as megakaryocyte progenitor cell clones.

### Analysis of Megakaryocyte Progenitor Cell Clones (First Round)

Each of the megakaryocyte progenitor cell clones obtained according to the method described above was washed twice using PBS, disseminated in a 6-well plate at 4 × 10⁵ cells/3 ml, and cultured in IMDM containing human SCF at 50 ng/ml, human TPO at 50 ng/ml, SR1 (Calbiochem) at 750 nM and 15% FBS. The supernatant was recovered 7 days later followed by evaluation of the number of platelets produced and platelet function. Evaluation of the number of platelets produced was carried out in the manner described below. Namely, fluorescent dye-bound antibodies to CD41 (BioLegend), CD42a (eBioscience) and CD42b (BioLegend) and propidium iodide (Sigma-Aldrich) were added to the culture supernatant and incubated for 30 minutes followed by analyzing using FACSVerseO (BD Biosciences). Analysis including excluding megakaryocyte progenitor cells based on size, followed by counting those cells positive for CD41, CD42a and CD42b and calculating as the number of platelets per megakaryocyte progenitor cell. Evaluation of platelet function was carried out in the manner described below. Namely, fluorescent dye-bound antibodies to CD41, CD42b and activated glycoprotein (GP) IIb/IIIa (PAC-1; BD Biosciences) and 0.4 mM phorbol 12-myristate 13-acetate (PMA) (Sigma-Aldrich) were added to the culture supernatant and incubated for 30 minutes followed by analyzing using FACSVerseO. In the analysis, the expression level of activated GP IIb/IIIa was used for evaluation by measuring as fluorescence intensity (MFI).

As a result of evaluating platelet production volume and platelet function for 22 megakaryocyte progenitor cell clones according to the method described above, platelet production volume was confirmed to be 1.6 times to 1.9 times higher than the control (megakaryocyte progenitor cells prior to cloning) for clone 1, clone 4 and clone 13 (Fig. 1A). With respect to platelet function, the highest level of activity was demonstrated by clone 13 and was indicated to react well to PMA stimulation in comparison with the control (Fig. 1B).

### Analysis of Megakaryocyte Progenitor Cell Clones (Second Round)

The five clones (1, 2, 4, 11 and 13) that demonstrated high platelet production volumes and platelet function in the results for the first round of analysis were re-analyzed in the same manner. Platelet production volume was confirmed to be about 1.3 times higher than the control for clone 4 only (Fig. 2A). In addition, although platelet function was confirmed to be about 3.7 times higher than the control for clone 13, results for the platelet function of clones 1, 2, 4 and 11 differed from the results of the first round of analysis in that there was no change from the control (Fig. 2B).

### Analysis of Megakaryocyte Progenitor Cell Clones (Third Round)

Analyses were conducted again in the same manner for the results of the second round of analysis. There were no changes in platelet production volume with respect to the control (Fig. 3A). In addition, although platelet function was confirmed to be 1.7 times and 1.6 times higher, respectively, than the control for clones 4 and 13, the differences were small (Fig. 3B).

According to these results, megakaryocyte progenitor cell clones obtained by the limiting dilution method were confirmed to not demonstrate stable function with respect to platelet production capacity and the platelets produced. Thus, use of the limiting dilution method was suggested to be unsuitable for cloning of megakaryocyte progenitor cells.

### Example 1

### Cloning of Megakaryocyte Progenitor Cells by Reprogramming

iPS cells (secondary iPS cells) were prepared by reprogramming megakaryocyte progenitor cells obtained with the previously described method followed by carrying out cloning with the secondary iPS cells and again inducing the differentiation of the cells into megakaryocyte progenitor cells to clone these cells (secondary megakaryocyte progenitor cells) (Fig. 4A). The following provides a detailed description thereof.

After introducing four types of episomal vector plasmids (pCXLE-hOCT3/4-shp53-F, pCXLE-hSK, pCXLE-hUL and pCXWB-EBNA1; Okita K, et al., Stem Cells. 31:458-66, 2013 and Okita K, et al., Nat Methods. 8:409-12, 2011) into 1 × 10⁶ cells of a primary cultured megakaryocyte progenitor cell line by electroporation using Amaxa Nucleofector, the cells were disseminated at 3 × 10⁵ cells/dish in feeder cells in the form of MEF at 1-2 × 10⁵ cells/6 cm dish. The resulting colonies were picked from the dish 14 days later and subjected to expansion culturing, after which 10 secondary iPS cell clones were used in analysis.

### Analysis of Secondary iPS Cell Clones

Genomic DNA was extracted from the 10 secondary iPS cell clones obtained according to the method described above, and quantitative PCR was carried out on c-MYC and BMI1 within exons using primers compatible with the PCR reaction. As a result, an examination of the number of insertions of exogenous c-MYC and BMI1 other than the two intrinsic copies revealed that the number of insertions of exogenous BMI1 of the 10 types of secondary iPS cell clones was about 7 copies to 26 copies, while the number of insertions of exogenous c-MYC was about 1 copy to 6 copies, and were all different (Fig. 4B).

Genomic DNA was extracted from the 10 secondary iPS cell clones (using some clones that were different from those previously described) obtained according to the method described above followed by analyzing all of the genome sequences, and when the number of insertions of exogenous c-MYC and BMI1 were examined, they were confirmed to be inserted into chromosomes at the ratios indicated in the following Table 1. The secondary iPS cell clones were confirmed to contain about 3 times to 5 times the number of insertions of BMI1 in comparison with the number of insertions of c-MYC.

**[Table 1]**

| | BMI/MYC |
|---|---|
| 2nd-iPS_1 | 3.90 |
| 2nd-iPS_4 | 2.89 |
| 2nd-iPS_11 | 4.97 |
| 2nd-iPS_13 | 3.47 |
| 2nd-iPS_14 | 3.97 |
| 2nd-iPS_15 | 2.95 |
| 2nd-iPS_16 | 4.75 |
| 2nd-iPS_19 | 2.94 |
| 2nd-iPS_20 | 4.27 |
| 2nd-iPS_22 | 4.89 |

According to the above results, although the number of incorporations of exogenous genes introduced in the step for producing megakaryocyte progenitor cells differed for each of the megakaryocyte progenitor cells, in the case of having been incorporated at a constant ratio, megakaryocyte progenitor cells were suggested to have been produced.

### Induction of Secondary Megakaryocyte Progenitor Cell Clones from Secondary iPS Cell Clones

HPC clones were respectively obtained through iPS-sac 14 days later from three secondary iPS cell clones (#4, #11 and #12) according to the previously described method. The resulting HPC clones were disseminated into 6-wells at 1 × 10⁵ cells/well followed by culturing for 27 days in EBM containing SCF at 50 ng/ml, TPO at 50 ng/ml and doxycycline at 0.5 µg/ml to obtain secondary megakaryocyte progenitor cell clones. Analysis of the resulting three megakaryocyte progenitor cell clones revealed that the clones were positive for CD41a and CD41b, negative for CD235, and were obtained in the form of a uniform cell group (Figs. 5A and 5B).

### Preparation of HLA-Deficient (HLA-null) Secondary iPS Cell Clones

The Target vector indicated in Fig. 6A was introduced into a secondary iPS cell clone (#11) obtained according to the method described above to delete Exon 1 of β2-Microglobulin (β2m) by homologous recombination. Whether or not homologous recombination was carried out properly was determined by confirming PCR products using primers designed at the sites indicated in Fig. 6A for the wild type and mutant (Fig. 6B). As a result, β2m-deficient secondary iPS cell clones were established for two clones (Clones 3 and 4).

Continuing, β2m-deficient secondary megakaryocyte progenitor cell clones were induced according to the previously described method. In addition, platelets were obtained from the supernatant according to the previously described method. When flow cytometry was carried out on the β2m-deficient secondary megakaryocyte progenitor cell clones and platelets using antibodies to β2m (BD Pharmingen) and HLA (BD Pharmingen), all were confirmed to be deficient in β2m and HLA (Fig. 7A).

### Confirmation of Platelet Function of β2m-deficient Secondary Megakaryocyte Progenitor Cell Clones

After stimulating platelets present in the culture supernatant of wild-type secondary iPS cell clone (#11) and β2m-deficient secondary megakaryocyte progenitor cell clones with PMA in the same manner as previously described, the clones were contacted with CD42a antibody, CD42b antibody and PAC1, and when the positive levels of CD42a, CD42b and PAC1 were measured and evaluated on the basis of fluorescence intensity (MFI), there were no differences observed between the wild type and β2m-deficient type (Fig. 7B). On the basis of the above, platelets deficient in β2m were indicated to have the same function as wild-type platelets.

### Example 2

### Search for iPS Cell Marker Suitable for Induction of Megakaryocyte Progenitor Cells

mRNA was extracted from two types of secondary iPS cells (to be referred to as Good-iPSC) able to establish imMKCL obtained according to the previously described method and from two types of iPS cells (to be referred to as Bad-iPSC) unable to establish imMKCL obtained according to the previously described method followed by averaging and analyzing the expression levels of the two types of Good-iPSC and Bad-iPSC with a microarray using the Illumina HumanHT-12 v4.0 or Affymetrix Gene Chip Human Gene 2.0 ST Array in accordance with the manuals provided by the manufacturers (Figs. 8A and 8B).

Moreover, hematopoietic progenitor cells (HPC) were induced from the Good-iPSC and Bad-iPSC using the same method as previously described (respectively referred to as Good-HPC and Bad-HPC) followed by analyzing with a microarray using the Illumina HumanHT-12 v4.0 or Affymetrix Gene Chip Human Gene 2.0 ST Array (Figs. 8A and 8B).

As a result of analyzing with the above-mentioned microarrays, MEG3 was confirmed to be highly expressed for both the Good-iPSC and Good-HPC. Thus, in secondary iPS cell clones and HPC induced therefrom, confirmation of expression of MEG3 was suggested to enable selection of secondary iPS cell clones suitable for induction of megakaryocyte progenitor cells.

### Example 3

### Proliferative Capacity of Megakaryocyte Progenitor Cells Derived from Secondary iPS Cell Clone

The cell proliferative capacity of megakaryocyte progenitor cells derived from a secondary iPS cell clone was compared with that of a megakaryocyte progenitor cell line prior to cloning in order to examine the effect of the cloning according to the present invention. In this experiment, a clone derived from 2nd-iPS_11 listed in Table 1 was used for the secondary iPS cell clone. More specifically, an HPC clone obtained from 2nd-iPS_11 through iPS-sac 14 days later according to the previously described method was disseminated in a 6-well dish at 1 × 10⁵ cells/well followed by culturing for 27 days in EBM containing SCF at 50 ng/ml, TPO at 50 ng/ml and doxycycline at 0.5 µg/ml to obtain a secondary megakaryocyte progenitor cell clone (Clone 2). Megakaryocyte progenitor cells (Parental) prior to cloning prepared in the section entitled "Production of Megakaryocyte Progenitor Cells" were used as a comparative example. These cells were disseminated in a 6-well dish at 1 × 10⁵ cells/well and cultured for 15 days in EBM containing SCF at 50 ng/ml, TPO at 50 ng/ml and doxycycline at 0.5 µg/ml.

As a result of periodically counting the number of cells during the 15 day culturing period, megakaryocyte progenitor cells derived from the secondary iPS cell clone clearly demonstrated more rapid growth than the megakaryocyte progenitor cells prior to cloning. The results are shown in Figs. 9A and 9B.

### Maturation Capacity of Megakaryocyte Progenitor Cells Derived from Secondary iPS Cell Clone

A secondary megakaryocyte progenitor cell clone (Clone 2) cultured for 15 days was cultured under conditions facilitating megakaryocyte maturation (including adding SCF at 50 ng/ml and TPO at 50 ng/ml to EBM followed by further adding SR-1 (StemRegenin1) (Selleckchem) at 750 nM and Y27632 (Wako) at 10 µM). Megakaryocyte progenitor cells (Parental) were also allowed to mature to megakaryocytes under the same culturing conditions as the secondary megakaryocyte progenitor cell clone. Images of both cells photographed by time-lapse imaging are shown in Fig. 10A, while the results of analyzing the resulting images with ImageJ are shown in Fig. 10B. On the basis of these results, megakaryocytes cloned in accordance with the present invention were shown to demonstrate superior maturation capacity (cellular hypertrophy) than megakaryocytes derived from the original clone cells.

### Platelet Production Capacity of Megakaryocyte Progenitor Cells Derived from Secondary iPS Cell Clone

The number of cells that formed platelets was measured visually in the images shown in Fig. 10A in order to compare platelet production capacity of the resulting megakaryocytes. As a result, megakaryocytes derived from cells cloned in accordance with the present invention clearly demonstrated superior platelet production capacity (number of proplatelets formed) (**: p<0.01).

## Claims

1. An in vitro method for producing a stem cell clone, which comprises the steps of:
(i) introducing, into stem cells capable of being induced to differentiate into megakaryocyte progenitor cells, an exogenous gene associated with induction of differentiation into megakaryocyte progenitor cells;
(ii) inducing differentiation of the stem cells, introduced with the exogenous gene, into the megakaryocyte progenitor cells;
(iii) dedifferentiating the differentiation-induced megakaryocyte progenitor cells; and
(iv) isolating stem cells having the exogenous gene incorporated into a chromosome thereof from a colony of the stem cells formed in step (iii);
wherein the exogenous gene associated with induction of differentiation is at least one exogenous gene selected from the group consisting of oncogenes including MYC family genes, genes (polycomb genes) inhibiting expression of a p16 gene or a p19 gene including Bmi1, and apoptosis suppressing genes including BCL-XL gene;
wherein the dedifferentiation in step (iii) is carried out by introducing a reprogramming factor selected from the group consisting of OCT3/4, SOX2 and KLF4 into the differentiation-induced megakaryocyte progenitor cells.

2. The method according to claim 1, wherein the differentiation induction efficiency of isolated stem cell clones into megakaryocyte progenitor cells is higher in comparison with that of stem cells prior to cloning.

3. The method according to claim 1 or claim 2, wherein stem cells expressing MEG3 are isolated.

4. The method according to any one of claims 1 to 3, wherein the exogenous gene associated with induction of differentiation is functionally linked to a drug-responsive promoter.

5. An in vitro method for producing megakaryocyte progenitor cells, which comprises the step of:
inducing differentiation of stem cell clones produced according to the method according to any of claims 1 to 4 into megakaryocyte progenitor cells.

6. An in vitro method for producing platelets, which comprises the steps of:
inducing differentiation of stem cell clones produced according to the method according to any of claims 1 to 4 into megakaryocyte progenitor cells; and
allowing the differentiation-induced megakaryocyte progenitor cells to mature into megakaryocytes and release platelets.

7. The method according to claim 6, wherein the produced platelets are deficient in HLA.

## Patentansprüche

1. In-vitro-Verfahren zum Produzieren eines Stammzellklons, das die folgenden Schritte beinhaltet:
(i) Einbringen eines exogenen Gens, das mit der Induktion der Differenzierung zu Megakaryozyten-Vorläuferzellen assoziiert ist, in Stammzellen, die zur Differenzierung zu Megakaryozyten-Vorläuferzellen induziert werden können;
(ii) Induzieren der Differenzierung der Stammzellen, in die das exogene Gen eingebracht wurde, zu den Megakaryozyten-Vorläuferzellen;
(iii) Dedifferenzieren der differenzierungsinduzierten Megakaryozyten-Vorläuferzellen; und
(iv) Isolieren von Stammzellen, bei denen das exogene Gen in ein Chromosom davon eingebaut wurde, von einer Kolonie der in Schritt (iii) gebildeten Stammzellen;
wobei das mit der Differenzierungsinduktion assoziierte exogene Gen wenigstens ein exogenes Gen ist, das ausgewählt ist aus der Gruppe bestehend aus Onkogenen, inkl. Gene der MYC-Familie, Gene (Polycomb-Gene), die die Expression eines p16-Gens oder eines p19-Gens inhibieren, inkl. Bmi1, und Apoptose-supprimierender Gene, inkl. BCL-XL-Gen;
wobei das Dedifferenzieren in Schritt (iii) durch Einbringen eines Reprogrammierungsfaktors, ausgewählt aus der Gruppe bestehend aus OCT3/4, SOX2 und KLF4, in die differenzierungsinduzierten Megakaryozyten-Vorläuferzellen erfolgt.

2. Verfahren nach Anspruch 1, wobei die Effizienz der Differenzierungsinduktion von isolierten Stammzellklonen zu Megakaryozyten-Vorläuferzellen im Vergleich zu der von Stammzellen vor dem Klonieren höher ist.

3. Verfahren nach Anspruch 1 oder Anspruch 2, wobei Stammzellen, die MEG3 exprimieren, isoliert werden.

4. Verfahren nach einem der Ansprüche 1 bis 3, wobei das mit der Differenzierungsinduktion assoziierte exogene Gen funktionsfähig mit einem auf Arzneimittel ansprechenden Promotor verknüpft ist.

5. In-vitro-Verfahren zum Produzieren von Megakaryozyten-Vorläuferzellen, das die folgenden Schritte beinhaltet:
Induzieren der Differenzierung von Stammzellklonen, die mit dem Verfahren nach einem der Ansprüche 1 bis 4 produziert wurden, zu Megakaryozyten-Vorläuferzellen.

6. In-vitro-Verfahren zum Produzieren von Blutplättchen, das die folgenden Schritte beinhaltet:
Induzieren der Differenzierung von Stammzellklonen, die mit dem Verfahren nach einem der Ansprüche 1 bis 4 produziert wurden, zu Megakaryozyten-Vorläuferzellen; und
Ermöglichen, dass die differenzierungsinduzierten Megakaryozyten-Vorläuferzellen zu Megakaryozyten reifen und Blutplättchen freisetzen.

7. Verfahren nach Anspruch 6, wobei die produzierten Blutplättchen HLA-defizient sind.

## Revendications

1. Procédé in vitro destiné à produire un clone de cellule souche, qui comprend les étapes suivantes:
(i) introduction, dans des cellules souches capables d'être induites pour se différencier en cellules progénitrices de mégacaryocytes, d'un gène exogène associé à l'induction de la différentiation en cellules progénitrices de mégacaryocytes;
(ii) induction de la différentiation des cellules souches, dans lesquelles le gène exogène a été introduit, en cellules progénitrices de mégacaryocytes;
(iii) dédifférenciation des cellules progénitrices de mégacaryocytes induites par différentiation; et
(iv) isolation de cellules souches ayant le gène exogène incorporé dans un chromosome de celles-ci à partir d'une colonie des cellules souches formées dans l'étape (iii);
dans lequel le gène exogène associé à l'induction de la différentiation est au moins un gène exogène choisi parmi le groupe constitué par des oncogènes incluant des gènes de la famille MYC, des gènes (gènes Polycomb) inhibant l'expression d'un gène p16 ou d'un gène p19 incluant Bmi1, et des gènes supprimant l'apoptose incluant un gène BCL-XL;
dans lequel la dédifférenciation dans l'étape (iii) est réalisée par l'introduction d'un facteur de reprogrammation choisi parmi le groupe constitué par OCT3/4, SOX2 et KLF4 dans les cellules progénitrices de mégacaryocytes induites par différentiation.

2. Procédé selon la revendication 1, dans lequel l'efficacité de l'induction de la différentiation de clones de cellules souches isolées en cellules progénitrices de mégacaryocytes est plus élevée par rapport à celle de cellules souches avant le clonage.

3. Procédé selon la revendication 1 ou la revendication 2, dans lequel des cellules souches exprimant MEG3 sont isolées.

4. Procédé selon l'une quelconque des revendications 1 à 3, dans lequel le gène exogène associé à l'induction de la différentiation est lié de manière fonctionnelle à un promoteur répondant à un médicament.

5. Procédé in vitro destiné à produire des cellules progénitrices de mégacaryocytes, qui comprend la méthode suivante:
induction de la différentiation de clones de cellules souches produits selon le procédé selon l'une quelconque des revendications 1 à 4 en cellules progénitrices de mégacaryocytes.

6. Procédé in vitro destiné à produire des plaquettes, qui comprend les étapes suivantes:
induction de la différentiation de clones de cellules souches produits selon le procédé selon l'une quelconque des revendications 1 à 4 en cellules progénitrices de mégacaryocytes; et
fait de laisser les cellules progénitrices de mégacaryocytes induites par différentiation se développer en mégacaryocytes et libérer des plaquettes.

7. Procédé selon la revendication 6, dans lequel les plaquettes produites sont dépourvues de HLA.
